# EUROPEAN PATENT SPECIFICATION

(11) **EP 1 711 224 B1**
(45) Date of publication and mention of the grant of the patent: **30.05.2007**
(21) Application number: 05702790.6
(22) Date of filing: 26.01.2005
(51) Int. Cl.: A61N 1/36, A61B 5/05

(54) **METHOD AND APPARATUS FOR SIMULATING TASTE SENSATIONS IN A TASTE SIMULATION SYSTEM**
VERFAHREN UND GERÄT ZUR SIMULATION VON GESCHMACKSEMPFINDUNGEN IN EINEM GESCHMACKSSIMULATIONSSYSTEM
PROCEDE ET DISPOSITIF SERVANT A SIMULER DES SENSATIONS DE GOUT DANS UN SYSTEME DE SIMULATION DE GOUT

(30) Priority: 27.01.2004 US 539781 P
(43) Date of publication of application: 18.10.2006
(73) Proprietor: Koninklijke Philips Electronics N.V., 5621 BA Eindhoven (NL)
(72) Inventor: BURGMANS, Tom, NL-5621 BA Eindhoven (NL)
(74) Representative: Groenendaal, Antonius W. M.
(86) International application number: PCT/IB2005/050303
(87) International publication number: WO 2005/072821

(56) References cited:
- WO-A-02/060522
- US-A- 4 112 596
- US-A- 4 570 636
- US-A- 4 629 424
- US-A- 4 940 056
- Physiological Measurement IOP Publishing UK, vol. 20, no. 4, 1999, pages 385-400, XP002325459 ISSN: 0967-3334

## Description

This invention relates generally to sensory stimulation, and more particularly, to the production of taste sensations on a human tongue using electronic stimuli.

To the best of the inventor's knowledge, there are no known prior art patents that are directed to electronically stimulating the tongue palate to simulate various taste sensations. Other measurements have been performed including receiving signals from the tongue.

U.S. Pat. No. 5,212,476 issued to Sean R. Maloney describes an intra-oral device for detecting electromyographic (EMG) signals from the tongue. The Maloney device describes a single splint having a convex side which may be in contact with either the maxillary or mandibular and includes active electrodes mounted on the splint adjacent to the tongue. Muscle contraction causes the movement of charged ions across membrane and myoneural junctions thereby establishing electric fields of varying intensities depending on the degree of muscle contraction. These electric fields, i.e., (EMG) signals, may be detected by electrodes which commonly are placed on the skin or inserted into the muscle tissue in the form of a needle. These signals provide useful information to the physician with respect to the neuromuscular condition.

US-A-4570636 discloses a method for simulating taste sensations by electrically stimulating a human tongue, and a device for carrying out the method.

The present invention provides a tongue plate device that enables a user (wearer of the device) to receive electronic stimulation in various regions of the user's tongue to simulate the different taste sensations (i.e., sweet, sour, salty, bitter), that are conventionally stimulated via chemical means. The tongue plate device includes means for measuring the saliva response of the user to determine the user's preference or lack thereof for the various taste sensations being simulated. The tongue plate device is sized to fit over a human tongue and includes a lower surface configured to lie on top of a human tongue, the lower surface including a plurality of electronic stimulation nodes operable to transmit externally generated electrical signals to stimulate sensory nerve fibers within the human tongue at predetermined locations; and an upper surface including saliva flow measurement means for measuring a rate of change in the saliva flow rate of a human in response to the stimulation of the sensory nerve fibers.

Systems according to the present invention generally include a tongue plate apparatus having a plurality of stimulation nodes, each node coupled, via wired or wireless means, to an interactive device configured to receive tongue plate data from a content provider, process the tongue plate data and provide the processed tongue plate data to the tongue plate to excite the plurality of stimulation nodes. The tongue plate apparatus also includes saliva flow rate measuring means for measuring the saliva flow rate of a user in response to the applied electronic taste stimulation signals. The interactive device is also configured to receive feedback data from the saliva flow rate measuring means of the tongue plate. The feedback data is capable of being stored and analyzed to determine a user's taste preferences.

Referring now to the drawings in which like reference numbers represent corresponding parts throughout, where:
FIG. 1 is a simplified illustration of a cross-sectional view of a human tongue;
FIG. 2 is a perspective view of a taste sensation system according to an embodiment of the invention;
FIGS. 3a and 3b are enlarged views of the stimulation nodes of FIG. 2, according to various embodiments of the invention;
FIG. 4a-c are illustrations of the saliva flow measuring system of FIG. 2; and
FIG. 5 is of a method a flowchart according to one embodiment of the present invention.

In the following description of the specific embodiments, reference is made to the accompanying drawings which form a part hereof and which show by way of illustration the specific embodiments in which the invention may be practiced. In the accompanying drawings, like reference numbers represent corresponding parts throughout the several views. It is to be understood that other embodiments may be utilized as structural changes may be made without departing from the scope of the invention.

It is instructive to first briefly review some of the functionality and structure of the human tongue. Humans perceive all taste qualities all over the tongue. There may be increased sensitivity to certain qualities in certain areas. In addition, our taste system provides information on the intensity and pleasantness (or unpleasantness) of taste as well. The tongue is made up of muscles covered by mucous membranes. There are very small nodules, called papillae, from the top surface of the tongue, which give it its rough texture. Between the papillae at the sides and base of the tongue are small, bulblike structures that are sensory organs, called "taste buds," which enable us to enjoy the sensations of flavor and warn us when food is unfit to eat. The so-called "taste-buds" number somewhere between 5,000 to 10,000, scattered over the upper and side surfaces of the tongue. The taste buds are capable of chemically detecting each of the various taste sensations, however, each taste bud is more acutely attuned to a particular taste sensation.

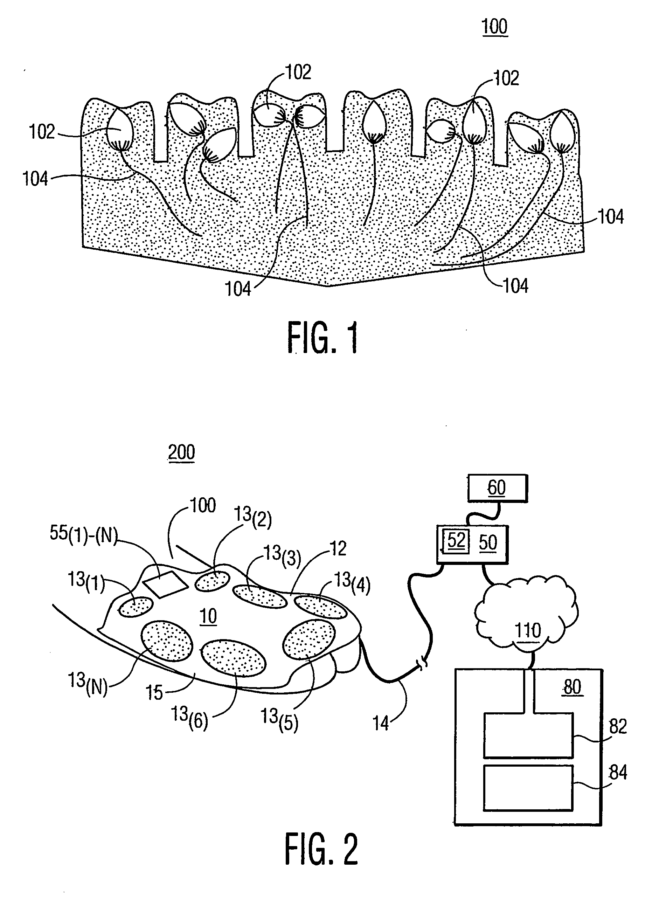

FIG. 1 is a simplified illustration of a cross-sectional view of a tongue 100 including taste buds 102, located in the upper surface of the tongue 100. Each taste bud 102 in the tongue 100 is coupled to the nervous system via the sensory nerve fibers 104. A key feature of the invention is the recognition by the inventor that producing taste sensations on the tongue 100 via conventional chemical means may be by-passed by the present invention in favor of direct electronic stimulation of the sensory nerve fibers 104 which couple the taste buds 102 of the tongue 100 to the nervous system (not shown).

Referring now to FIG. 2, an exemplary taste simulation system 200 for simulating the different taste sensations (i.e., sweet, sour, salty, bitter) in a human mouth will now be described in detail. As shown, taste simulation system 200 includes a tongue plate 10 connected to an interactive device 50 which is in turn connected to a content provider 80 via a broadcast network 110. The interactive device 50 is directly coupled to an external multimedia device 60.

The tongue plate 10 of FIG. 2 generally follows the contours of the wearer's tongue 100 and is configured to include a left and right shoulder 12, 15 that extend along the sides of the tongue. These shoulders 12, 15 serve to position the tongue plate 10 on and around the tongue and to prevent lateral slippage. The tongue plate 10 may be formed, if desired, with rounded edges and surfaces to prevent injury to the soft tissue of the tongue. The tongue plate 10 may be formed from one or more materials selected for their low cost and/or disposability. In addition, the tongue plate 10 may be pre-sterilized and packaged.

Embedded within the tongue plate 10 are a plurality of electronic stimulation nodes (13(1), 13(2), ..., 13(N)) positioned at various points on the tongue plate 10 operable to transmit externally generated electrical signals to stimulate sensory nerve fibers 104 within the human tongue 100 at predetermined locations. The stimulation nodes 13 are configured to receive electronic stimulation signals (e.g., currents and/or voltages) from the interactive device 50, coupled to the tongue plate 10, arranged externally of the user's body.

An upper surface of the tongue plate 10 includes saliva flow measurement means 55 for measuring a rate of change in the saliva flow rate of a human in response to the stimulation of the sensory nerve fibers 104.

In operation, in accordance with an application of the invention, tongue plate data is generated at a remote content provider 80 and is transmitted along with conventional broadcast content (which may be stored content 84), via content server 82, over broadcast network 110, to the interactive device 50 which translates the tongue plate data into a plurality of electronic stimulation signals to be applied to the tongue plate 10. The conventional broadcast content may be displayed to a user wearing the tongue plate 10 on an external multimedia device 60 synchronously with the electronic stimulation signals being applied to the tongue plate 10. In this manner, a user may experience simulated taste sensations on his (her) tongue 100 in coordination with a multimedia presentation (e.g., cooking program) being presented to the user on the external multimedia device 60. The saliva flow measurement means 55 on the tongue plate feeds back saliva flow rate information from the user in response to the electronic stimulation signals which may be stored in a memory 52 of the interactive device 50 and/or transmitted back to the content provider 80. The saliva flow rate information effectively constitutes taste preferences of the user.

In each of the embodiments to be described herein, electronic stimulation of the wearer's tongue 100 is effected from electronic signals provided by the interactive device 50 supplied to the stimulation nodes 13 each of which comprise one or more pole groups 20(a),..., 20(n).

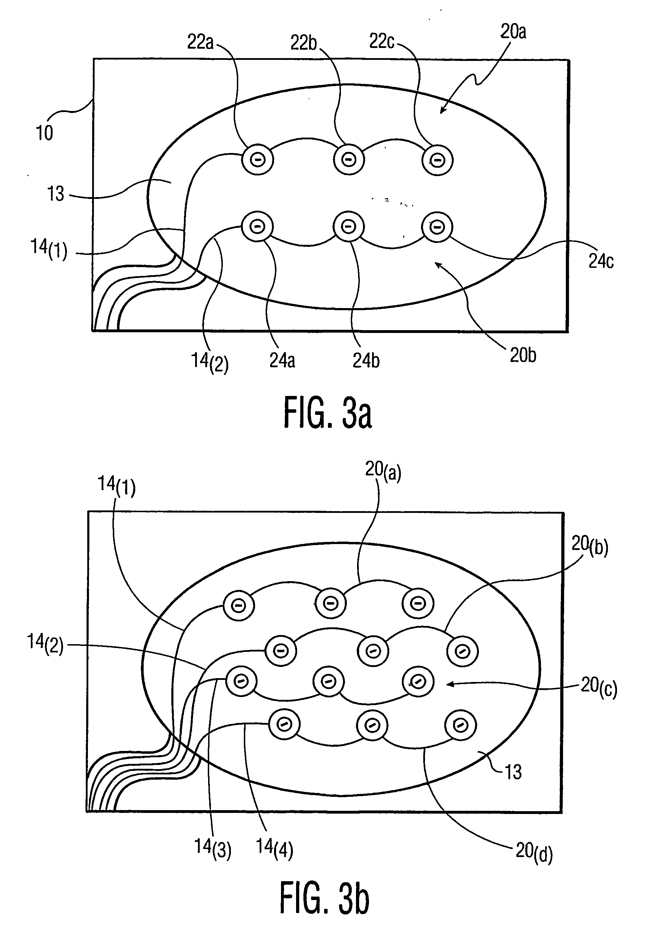

FIGS. 3a and 3b are more detailed illustrations of the stimulation nodes 13 of FIG. 2, according to various embodiments.

FIG. 3a is an illustration of one stimulation node 13 of FIG. 2 according to an embodiment of the invention. The stimulation node 13 is shown to include two pole groups 20a, 20b. Each pole group 20a, 20b is electrically isolated from the other pole groups. Further, each pole group 20a, 20b is independently coupled to separate channels of the interactive device (50) via cabling (i.e., pole group 20a coupled via pole group lead wire 14(1) and pole group 20b coupled via a pole group lead wire 14(2)).

With reference first to the embodiment of FIG. 3a, each pole group 20a, 20b is shown to be respectively comprised of three stimulation nodes 22a-c, 24a-c. It should be appreciated that different embodiments may include more or less stimulation nodes 22, 24 per pole group. Each stimulation node 22, 24 in FIG. 3 is configured to include a negative (-) inner region encapsulated by an outer positive (+) region for forming a complete circuit. Other embodiments may invert the node configuration. That is, the stimulation node 22, 24 may be otherwise configured as having a positive (+) inner region encapsulated by a negative (-) outer region.

Each stimulation node 22,24 in the pole group is capable of transmitting an electric stimulation signal (e.g., current and/or voltage) to electronically stimulate one or more sensory nerve fibers 104 in the immediate vicinity of the tongue 100 over which the stimulation nodes 22, 24 of each pole group is situated (see Figure 1). Each pole group 20a, 20b receives a unique and separate electronic stimulation signal from the interactive device 50 via a corresponding pole group lead wire 14(1), 14(2).

FIG. 3b is an illustration of a stimulation node 13 according to another embodiment of the invention. As shown, the stimulation node 13 is comprised of four pole groups, i.e., 20(a) -20(d). Additional pole groups (e.g., 20(c), 20(d)) may provide heightened sensitivity in certain areas of the tongue 100 where it is desired. Of course, it will be recognized that the number and configuration of pole groups are a design matter. For example, it is contemplated that in certain embodiments, pole groups 20 may intersect and/or overlap at certain points.

It is to be appreciated that the tongue plate 10 may also communicate with the interactive device 50 in a wireless manner. Thus, the tongue plate 10 may include a suitable wireless interface. The advantage of a wireless communications link is that it obviates the need for the wire leads 14 which may become entangled, dislodged or disconnected. Due to the fact that the tongue plate 10 communicates in a wireless manner, it will be appreciated that the tongue plate 10 carries its own power supply (not shown) which may be a battery-operated power supply.

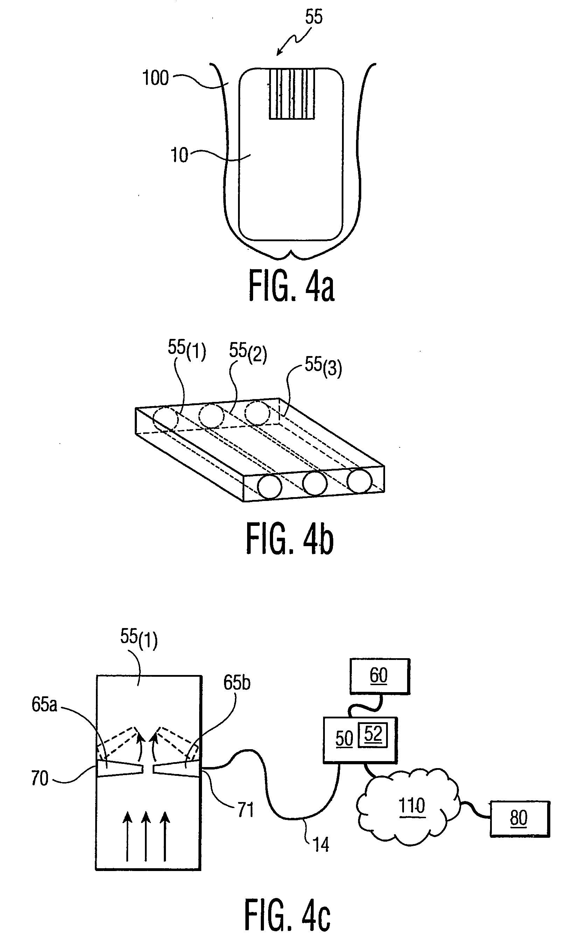

FIG. 4a is an illustration of a top view of the saliva flow measuring system 55 of the tongue plate 10 of FIG. 2 for measuring the saliva flow of a user in response to electronic stimulation of the tongue plate 10. As shown, the saliva flow measuring system 55 is located in a rear top portion of the tongue plate 10. It should be appreciated that each user's saliva response to the electronic stimulation signals will differ from other user's saliva response so that the taste response (i.e., saliva flow rate) for that user is unique to the user.

FIG. 4b is a more detailed illustration of the saliva flow measuring system 55, according to one embodiment of the invention. The saliva flow rate may be measured in one way by a set of parallel tube-like structures, referred to herein as pipes 55(1), 55(2), ..., 55(N) located parallel to the longitudinal axis of the tongue plate 10 through which saliva passes en route to the gullet. The pipes 55 have openings on both the proximal and distal ends for receiving a user's saliva and directing the saliva flow past the tongue palate en route to the user's gullet.

FIG. 4c is an internal view of one of the pipes 55(1) of FIG. 4b coupled via pole group lead wire 14(1) to an exemplary taste simulation system 200. The pipe 55(1) is shown to include a pair of hinged gates 65a, 65b, i.e., i.e., a left hinged gate 65a and a right hinged gate 65b, serve to modulate the saliva flow as it passes through the pipe 55(1). The two gates, 65a, 65b, (shown in both the open and closed positions) are pivotable about respective gate axes 70, 71. Those of ordinary skill in the art will recognize that a variety of other one-way gate type devices could also be used in place of the hinged gate pair 65a, 65b shown in the exemplary embodiment.

An output signal generated by the saliva flow measuring system 55 may be fed back to the content provider 80, via the interactive device 50, to measure the user's preference or lack thereof for the electronically stimulated taste sensation. The output signal could also be stored in a memory 52 associated with the interactive device 50.

The interactive device 50 includes at a minimum, hardware and/or software means for translating tongue plate information provided by the content source 80 into electrical signals suitable for use with the tongue plate 10. The interactive device 50 preferably includes a software application adapted to process tongue-plate information received from the content source 80. The tongue plate information may comprise, for example, which stimulation nodes 13 to excite on the tongue plate 10, the voltage and or current stimulation levels to be applied to the stimulation nodes 13, and various characteristics of the stimulation signal (e.g., AC/DC, block-wave, sine-wave, the signal duration and so on). The tongue plate information is processed by the interactive device 50 to provide output control signals to be provided to the tongue plate 10. Interactive device 50 can also include other components which are not illustrated in this example for simplicity purposes. For instance, interactive device 50 can include a user interface application and user interface lights, buttons, controls, etc. to facilitate viewer interaction with the device.

The external multimedia device 60, shown coupled to the interactive device 50, may be embodied as various devices, including an external set-top box, a television receiver including an integrated set-top box, a personal, laptop or handheld computer, and so on.

In an embodiment where the external multimedia device 60 is embodied as a set-top box, the external multimedia device 60 may be programmed to store user identification information, thereby obviating the requirement for the user to enter the information, or the set-top box may support entry of identification information.

As shown in FIG. 2, the content provider 80 includes a content server 82 and stored content 84, such as movies, television programs, commercials, music, and similar audio and/or video content. Content server 82 controls distribution of the stored content 84from content provider 80 to the interactive device 50 via broadcast network 110. Additionally, content server 82 controls distribution of live content (e.g., content that was not previously stored, such as live feeds) and/or content stored at other locations in the broadcast network 110. The content provider 80 may be embodied as a television broadcast station, a cable provider, a direct broadcast satellite, an Internet site that transmits content in a unicast, multicast, or broadcast fashion, a digital versatile disk player (DVD), a video cassette recorder (VCR), combinations of the above and other sources of content.

In the case where the content provider 80 is embodied as a television broadcast station, and the broadcast signal is a digital broadcast signal, the stimulation signals could be delivered from the content provider 80 to the interactive device 50 inside xlet packages which may be transmitted along with the conventional broadcast service. As is well known in the art, an xlet is a procedural application which a digital television (DTV) extension of an applet. An x-let is an interactive program that can be executed while watching/listening to the video/audio portions of the broadcast.

In the case where the content provider 80 is embodied as a television broadcast station, and the broadcast signal is an analogue broadcast signal, the stimulation signals could be delivered from the content source to the interactive device 50 in an inaudible portion of the audio spectrum, preferably outside the 20Hz - 20KHz range, so as not to interfere with the audible portion of the broadcast. Alternatively, the stimulation signals could be delivered from the content source to the interactive device 50 in an un-displayed portion of the video spectrum or via a teletext page.

In the case where the content provider 80 is embodied as a source other than a television broadcast station, such as, for example, an Internet site that transmits content in a unicast, multicast, or broadcast fashion, a digital versatile disk player (DVD), a video cassette recorder (VCR) and other similar sources of content, the stimulation signals could be delivered to the interactive device 50 via a suitable interface to the tongue plate 10.

It is also possible to have a separate telephone connection via an embedded modem, which can also be used as a return channel, for example, through an always-on return channel, such as a DOCSIS modem.

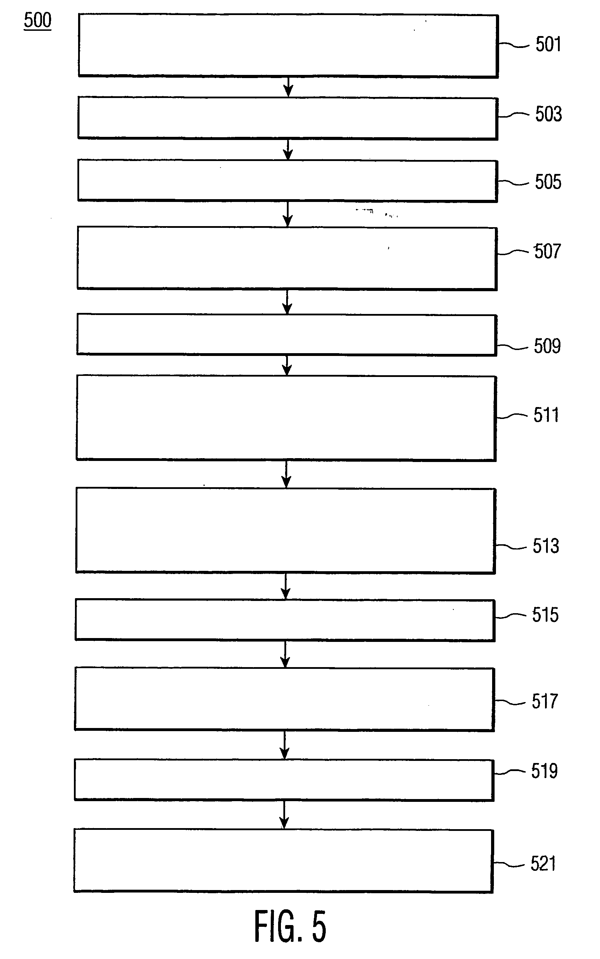

FIG. 5 is a flowchart of one embodiment of the present invention of a method 500 for providing sensory stimulation to a user's tongue in the context of an interactive broadcast directed to the preparation of various food items. Interactive broadcasts are well known, however, the invention provides a novel variation of an interactive broadcast in that the tongue plate 10 of the invention provides an interactive sensory stimulus of the food items being prepared during the broadcast. In the acts to be described, it is presumed that a user is tuned to a broadcast channel showing an interactive cooking program in which various dishes are prepared by a host, in a particular cooking style.

At Act 501: the process starts when either a user's external multimedia device 60 which for illustrative purposes is embodied as a television receiver including an integrated set-top box, is tuned to a channel displaying a cooking program. The cooking program typically includes the preparation of at least one recipe.

At Act 503: a user switches on the interactive device 50 coupled to the tongue plate 10.

At Act 505: The, user attempts to log in to the interactive device 50 by entering his(her) username and password, which have previously been provided to the user, in appropriate data fields and clicking on a "Log In" icon. For a new user, the interactive device 50 solicits various information from the user and a username and password are provided once the user has provided this information. Alternatively, the user information may be pre-stored in a memory 52 associated with the interactive device 50, enabling the user to enter directly into the system with a single sign-on and eliminating the need for the user to log in each time the system 200 is accessed.

At Act 507: Once the user has successfully logged into the system, user preferences, such as preferred taste preferences, are loaded from a memory coupled to, or integrated within, the interactive device 50 based on a user identification set for that user.

At Act 509: The user inserts the tongue plate 10 into the user's mouth.

At Act 511: As the food items are being prepared on the cooking program, control signals (tongue plate data) are transmitted along with conventional audio/video information from a broadcast station 80 to the interactive device 50.

At Act 513: The interactive device 50 processes the control information to generate appropriate electronic stimulation signals for stimulating appropriate stimulation nodes 13 on the tongue plate 10 associated with the food item being prepared. The stimulating signals are coordinated with the visual/audio signals being delivered to the television set 25.

At Act 515: The saliva flow rate is measured in response to the stimulation signals and appropriate feedback signals are generated corresponding to the measured saliva flow rate and stored in the memory 52 of the interactive device 50.

At Act 517: The feedback information is transmitted back to the broadcast station 80 via a return channel. In alternate embodiments, the feedback information can be used in a variety of ways, including, but not limited to:
- market information for food companies.
- Provide the host of the cooking program with an indication of preferred items, thus allowing the host to continue cooking with the preferred items.
- stored inside the interactive device 50 in a directory identifying the user, e.g., "good tasting flavors for user X".
- The feedback information could be used as input for other applications such as games, other x-lets or even for application running on other devices (e.g. computer) then the said interactive device.

At Act 519: The user removes the tongue plate 10 to be washed and dried.

At Act 521: The user logs off from the interactive device 50 and switches off the interactive device.

Although this invention has been described with reference to particular embodiments, it will be appreciated that many variations will be resorted to without departing from the scope of this invention as set forth in the appended claims. The specification and drawings are accordingly to be regarded in an illustrative manner and are not intended to limit the scope of the appended claims.

## Claims

1. A tongue plate (10) apparatus sized to fit over a human tongue (100), the tongue plate (10) including:
a lower surface including a plurality of electronic stimulation nodes (13) operable to transmit externally generated electrical signals to stimulate sensory nerve fibers (102) within the human tongue (100) at predetermined locations; and
an upper surface including saliva flow measurement means (55) for measuring a rate of change in the saliva flow rate of a human in response to the stimulation of the sensory nerve fibers (102).

2. The tongue plate (10) of Claim 1, wherein said tongue plate (10) further includes a left and right shoulder (12, 14) extending along the sides of the human's tongue, said left and right shoulders (12, 14) serving to prevent lateral slippage and generally follow the contours of the human's tongue.

3. The tongue plate (10) of Claim 1, wherein the tongue plate (1) is formed with rounded edges and surfaces to prevent injury to the soft tissue of the human's tongue.

4. The tongue plate (10) of Claim 1, wherein said electrical signals are one of AC signals, DC signals and a combination of AC and DC signals.

5. The tongue plate of Claim 1, wherein said saliva flow measurement means (55) is a plurality of parallel pipes (55(1) - 55(N)) positioned in a rear portion of said upper surface of said tongue plate (10) and extend horizontally along a line parallel to a longitudinal axis of said tongue plate (10) such that at least a portion of a saliva flow produced in response to said electrical stimulation flows through said parallel pipes (55(1) - 55(N)).

6. The tongue plate (10) of Claim 5, wherein an interior region of each of said parallel pipes (55(l)-55(N)) includes a left hinged gate (65a) and a right hinged gate (65b), working cooperatively and wherein each gate 65a, 65b is pivotable about respective left and right gate axes (70, 71) allowing said gates 65a, 65b to rotate from a first closed position to a second outwardly extending position.

7. The tongue plate (10) of Claim 1, wherein each of the plurality of stimulation nodes (13) are arranged in pole groups (20), wherein each pole group (20) includes one or more stimulation nodes (22, 24).

8. The tongue plate (10) of Claim 1, wherein each stimulation node (22, 24) is comprised of a negative inner region encapsulated by an outer positive region for conducting said electrical stimulation.

9. The tongue plate (10) of Claim 1, wherein each stimulation node (22) is comprised of a positive inner region encapsulated by an outer negative region for conducting said electrical stimulation.

10. A system for electronically simulating taste sensations in a user's tongue, the system comprising:
a content provider (80) operable to generate tongue plate data;
an interactive device (50) in communication with the content provider (80) and configured to receive the tongue plate data therefrom, convert the tongue plate data into electronic stimulation signals and output the electronic stimulation signals to a tongue plate (10); and
the tongue plate (10) configured to receive the electronic stimulation signals from the interactive device (50) and apply the electronic stimulation signals to stimulate sensory nerve fibers (102) within the tongue (100) of a human via a plurality of stimulation nodes (13) embedded within said tongue plate (10).

11. The system of Claim 10, wherein the content provider (80) includes a content server (82), said tongue plate data and stored/retrieved content (84) such as movies, television programs, commercials, music, and similar audio and/or video content, wherein said tongue plate data is synchronously supplied along with said stored/retrieved content (84) to said interactive device (50).

12. The system of Claim 10, wherein said interactive device (50) includes an output connector for connecting electrode lead-wires to specific ones of the plurality of stimulation nodes (13) included in said tongue plate (10).

13. The system of Claim 10, wherein said interactive device (50) is wirelessly coupled to said tongue plate (10).

14. The system of Claim 10, wherein said interactive device (50) further comprises memory means (52).

15. The system of Claim 10, wherein the tongue plate (10) further includes saliva flow rate measuring means (55) for measuring the saliva flow rate of a human.

16. The system of Claim 10, wherein the plurality of stimulation nodes (13) are arranged in pole groups (20) and each pole group (20) is comprised of one or more stimulation nodes (22, 24).

17. The system of Claim 10, wherein each pole group (20) is independently coupled to a separate channel of the interactive device (50).

18. A method for simulating taste sensations, comprising:
a) positioning a tongue plate (10) onto a tongue (100) of a user;
b) receiving, from an external source, a plurality of electronic stimulation signals at said tongue plate (10);
c) applying the received electronic stimulation signals to a plurality of stimulation nodes (13) disposed within said tongue plate (10) to electronically stimulate sensory nerve fibers (104) in said user's tongue (100); and
d) measuring a saliva flow rate of said user in response to said applied electronic stimulation signals.

19. The method of Claim 18, wherein said plurality of stimulation nodes (13) are configured to contact predetermined locations of an upper surface of the user's tongue (100).

20. The method of Claim 18, wherein the receiving act (b) further comprises the acts of:
transmitting, from a content provider (80), multimedia data including tongue plate data over a network (110) to an interactive device (50);
converting, at said interactive device (50), the transmitted tongue plate data into said electronic stimulation signals; and
providing said electronic stimulation signals to said tongue plate (10).

## Patentansprüche

1. Zungenplatteneinrichtung (10), derart bemessen, dass sie auf die menschliche Zunge (100) passt, wobei die Zungenplatte (10) Folgendes umfasst:
- eine untere Fläche mit einer Anzahl elektronischer Stimulationsknoten (13), wirksam zum Übertragen extern erzeugter elektronischer Signale zum Stimulieren sensorischer Nervenfasern (102) innerhalb der menschlichen Zunge (100) an vorbestimmten Stellen; und
- eine obere Fläche mit Speicherflussmessmitteln (55) zum Messen einer Änderungsrate in der Speicherflussrate eines Menschen in Reaktion auf die Stimulation der sensorischen Nervenfasern (102).

2. Zungenplatte (10) nach Anspruch 1, wobei die genannte Zungenplatte (10) weiterhin eine linke und eine rechte Schulter (12, 14) aufweist, die sich längs der Seiten der menschlichen Zunge erstrecken, wobei die genannte linke und rechte Schulter (12, 14) dazu dienen, eine laterale Schlüpfrigkeit zu vermeiden und im Allgemeinen den Umrissen der menschlichen Zunge zu folgen.

3. Zungenplatte (10) nach Anspruch 1, wobei die Zungenplatte (10) mit abgerundeten Kanten und Flächen gebildet ist um Verletzung des weichen Gewebes der menschlichen Zunge zu vermeiden.

4. Zungenplatte (10) nach Anspruch 1, wobei die genannten elektrischen Signale AC-Signale, DC-Signale oder eine Kombination von AC- und DC-Signalen sind.

5. Zungenplatte nach Anspruch 1, wobei die genannten Speichelflussmessmittel (55) eine Anzahl paralleler Röhrchen (55(1)-55(N)) sind, die in einem hinteren Teil der genannten oberen Fläche der genannten Zungenplatte (10) vorgesehen sind und sich horizontal längs einer Linie parallel zu der Längsachse der genannten Zungenplatte (10) derart erstrecken, dass wenigstens ein Teil eines in Reaktion auf die genannte elektrische Stimulation erzeugten Speichelflusses durch die genannten parallelen Röhrchen (55(I)-55(N)) fließt.

6. Zungenplatte (10) nach Anspruch 5, wobei ein inneres Gebiet jedes der genannten parallelen Röhrchen (55(1)-55(N)) eine linke gelenkige Sperre (65a) und eine rechte gelenkige Sperre (65b) aufweist, die zusammenarbeiten und wobei jede Sperre 65a, 65b um eine linke bzw. rechte Sperrenachse schwenkbar ist, wodurch die genannten Sperren 65a, 65b aus einer ersten geschlossenen Lage in eine zweite sich nach außen erstreckende Lage drehen können.

7. Zungenplatte (10) nach Anspruch 1, wobei alle Stimulationsknoten (13) in einer Polgruppen (20) gegliedert sind, wobei jede Polgruppe (20) einen oder mehrere Stimulationsknoten (22, 24) aufweist.

8. Zungenplatte (10) nach Anspruch 1, wobei jeder Stimulationsknoten (22, 24) aus einem negativen inneren Gebiet, eingekapselt durch ein äußeres Gebiet, besteht, und zwar zum Leiten der genannten elektrischen Stimulation.

9. Zungenplatte (10) nach Anspruch 1, wobei jeder Stimulationsknoten (22) aus einem positiven inneren Gebiet besteht, das durch ein äußeres negatives Gebiet eingeschlossen ist, und zwar zum Leiten der genannten elektrischen Stimulation.

10. System zum elektronischen Stimulieren von Geschmackssensationen in der Zunge eines Benutzers. Wobei das System Folgendes umfasst:
- einen Inhaltsprovider (80), der wirksam ist zum Erzeugen von Zungenplattendaten,
- eine interaktive Anordnung (50) in Kommunikation mit dem Inhaltsprovider (80) und konfiguriert zum Empfangen der Zungenplattendaten von demselben, zum Umwandeln der Zungenplattendaten in elektronische Stimulationssignale und zum Ausliefern der elektronischen Stimulationssignale zu einer Zungenplatte (10); und
- die Zungenplatte (10), konfiguriert zum Empfangen der elektronischen Stimulationssignale von der interaktiven Anordnung (50) und zum Zuführen der elektronischen Stimulationssignale zum Stimulieren von sensorischen Nervenfasern (102) innerhalb der Zunge (100) eines Menschen über eine Anzahl in die genannte Zungenplatte (10) eingebetteter Stimulationsknoten (13).

11. System nach Anspruch 10, wobei der Inhaltsprovider (80) einen Inhaltsserver (82) aufweist, wobei die genannten Zungenplattendaten und der gespeicherte/erfasst Inhalt (84), wie Filme, Fernsehprogramme, Werbesendungen, Musik und ähnliche Audio und/oder Videoinhalt, wobei die genannten Zungenplattendaten synchron mit dem genannten gespeicherten/erfassten Inhalt (84) der genannten interaktiven Anordnung (50) zugeführt werden.

12. System nach Anspruch 10, wobei die genannte interaktive Anordnung (50) ein Ausgangsanschlusselement aufweist zum Verbinden Elektrodenverbindungsdrähte mit betreffenden Stimulationsknoten (13) in der genannten Zungenplatte (10).

13. System nach Anspruch 10, wobei die genannte interaktive Anordnung (50) mit der genannten Zungenplatte (10) drahtlos verbunden ist.

14. System nach Anspruch 10, wobei die genannte interaktive Anordnung (50) weiterhin Speichermittel (52) umfasst.

15. System nach Anspruch 10, wobei die Zungenplatte (10) weiterhin Speichelflussratenmessmittel (55) aufweist zum Messen der Speichelflussrate eines Menschen.

16. System nach Anspruch 10, wobei die vielen Stimulationsknoten (13) in Polgruppen (20) gegliedert sind und wobei jede Polgruppe (20) aus einem oder mehreren Stimulationsknoten (22, 24) besteht.

17. System nach Anspruch 10, wobei jede Polgruppe (20) unabhängig mit einem einzelnen Kanal der interaktiven Anordnung (50) gekoppelt ist.

18. Verfahren zum Stimulieren von Geschmackssensationen, wobei das Verfahren Folgendes umfasst:
a) das Positionieren einer Zungenplatte (10) auf eine Zunge (100) eines Benutzers,
b) das Empfangen einer Anzahl elektronischer Stimulationssignale an der genannten Zungenplatte (10), herrührend von einer externen Quelle,
c) das Zuführen der empfangenen elektronischen Stimulationssignale zu einer Anzahl innerhalb der Zungenplatte (10) vorgesehener Stimulationsknoten (13) zum elektronischen Stimulieren sensorischer Nervenfasern (104) in der Zunge (100) des Benutzers, und
d) das Messen einer Speichelflussrate des genannten Benutzers in Reaktion auf die genannten zugeführten elektronischen Stimulationssignale.

19. Verfahren nach Anspruch 18, wobei die genannte Anzahl Stimulationsknoten (13) dazu vorgesehen sind, vorbestimmte Stellen einer oberen Fläche der Zunge (100) des Benutzers zu kontaktieren.

20. Verfahren nach Anspruch 18, wobei der Empfangsvorgang (b) weiterhin die nachfolgenden Vorgänge umfasst:
- das, von einem Inhaltsprovider (80) aus, Übertragen von Multimediadaten mit Zungenplattendaten über ein Netzwerk (110) zu einer interaktiven Anordnung (50),
- das, an der genannten interaktiven Anordnung (50), Umwandeln der übertragenen Zungenplattendaten in die genannten elektronischen Stimulationssignale, und
- das Liefern der genannten elektronischen Stimulationssignale zu der genannten Zungenplatte (10).

## Revendications

1. Appareil sous la forme d'une plaque linguale (10) dimensionné de manière à être installé sur une langue humaine (100), la plaque linguale (10) comprenant :
une surface inférieure incluant une pluralité de noeuds de stimulation électronique (13) pouvant fonctionner de manière à transmettre des signaux électriques générés de manière externe afin de stimuler des fibres nerveuses sensorielles (102) à l'intérieur de la langue humaine (100) en des endroits prédéterminés, et
une surface supérieure comprenant des moyens de mesure de débit salivaire (55) destinés à mesurer une vitesse de modification du débit salivaire d'un être humain en réaction à la stimulation des fibres nerveuses sensorielles (102).

2. Plaque linguale (10) suivant la revendication 1, dans laquelle ladite plaque linguale (10) comprend en outre des épaulements gauche et droit (12, 14) qui s'étendent sur les côtés de la langue de l'être humain, lesdits épaulements gauche et droit (12, 14) servant à éviter un glissement latéral et à suivre généralement les contours de la langue de l'être humain.

3. Plaque linguale (10) suivant la revendication 1, dans laquelle la plaque linguale (1) est formée avec des bords et surfaces arrondis afin d'éviter de blesser les parties molles de la langue de l'être humain.

4. Plaque linguale (10) suivant la revendication 1, dans laquelle lesdits signaux électriques sont pris parmi des signaux de courant alternatif (AC), des signaux de courant continu (DC) et une combinaison de signaux AC et DC.

5. Plaque linguale (10) suivant la revendication 1, dans laquelle lesdits moyens de mesure de débit salivaire (55) sont une pluralité de conduites parallèles (55(1) à 55(N)) positionnées sur une partie arrière de ladite surface supérieure de ladite plaque linguale (10) et qui s'étendent horizontalement le long d'une ligne parallèle à un axe longitudinal de ladite plaque linguale (10), de sorte qu'au moins une partie d'un débit salivaire produit en réaction à ladite stimulation électrique s'écoule à travers lesdites conduites parallèles (55(1) à 55(N)).

6. Plaque linguale (10) suivant la revendication 5, dans laquelle une région intérieure de chacune desdites conduites parallèles (55(1) à 55(N)) comprend un volet articulé gauche (65a) et un volet articulé droit (65b) fonctionnant en coopération, et dans laquelle chaque volet 65a, 65b peut pivoter sur des axes de volet gauche et droit respectifs (70, 71) permettant auxdits volets 65a, 65b de tourner d'une première position fermée à une seconde position s'étendant vers l'extérieur.

7. Plaque linguale (10) suivant la revendication 1, dans laquelle chacun de la pluralité de noeuds de stimulation (13) est agencé dans des groupes polaires (20), chaque groupe polaire (20) comprenant un ou plusieurs noeuds de stimulation (22, 24).

8. Plaque linguale (10) suivant la revendication 1, dans laquelle chaque noeud de stimulation (22, 24) comprend une région intérieure négative encapsulée par une région extérieure positive afin de conduire ladite stimulation électrique.

9. Plaque linguale (10) suivant la revendication 1, dans laquelle chaque noeud de stimulation (22) comprend une région intérieure positive encapsulée par une région extérieure négative afin de conduire ladite stimulation électrique.

10. Système destiné à simuler par voie électronique des sensations gustatives dans la langue d'un utilisateur, le système comprenant :
un fournisseur de contenu (80) pouvant fonctionner de manière à générer des données de plaque linguale;
un dispositif interactif (50) en communication avec le fournisseur de contenu (80) et configuré de manière à recevoir les données de plaque linguale en provenance de celui-ci, à convertir les données de plaque linguale en signaux de stimulation électronique et à fournir les signaux de stimulation électronique à une plaque linguale (10), et
la plaque linguale (10) configurée de manière à recevoir les signaux de stimulation électronique provenant du dispositif interactif (50), et à appliquer les signaux de stimulation électronique de manière à stimuler les fibres nerveuses sensorielles (102) à l'intérieur de la langue (100) d'un être humain par le biais d'une pluralité de noeuds de stimulation (13) noyés dans la plaque linguale (10).

11. Système suivant la revendication 10, dans lequel le fournisseur de contenu (80) comprend un serveur de contenu 82, lesdites données de plaque linguale, et le contenu stocké/extrait (84) comme des films, des programmes télévisés, des messages publicitaires, de la musique, et un contenu audio et/ou vidéo similaire, lesdites données de plaque linguale étant fournies de manière synchrone conjointement audit contenu stocké/extrait (84) audit dispositif interactif (50).

12. Système suivant la revendication 10, dans lequel ledit dispositif interactif (50) comprend un connecteur de sortie destiné à connecter des fils d'électrode à des noeuds spécifiques parmi la pluralité de noeuds de stimulation (13) inclus dans ladite plaque linguale (10).

13. Système suivant la revendication 10, dans lequel ledit dispositif interactif (50) est relié par un couplage sans fil à ladite plaque linguale (10).

14. Système suivant la revendication 10, dans lequel ledit dispositif interactif (50) comprend en outre des moyens de mémoire (52).

15. Système suivant la revendication 10, dans lequel la plaque linguale (10) comprend en outre des moyens de mesure de débit salivaire (55) destinés à mesurer le débit salivaire d'un être humain.

16. Système suivant la revendication 10, dans lequel la pluralité de noeuds de stimulation (13) sont agencés dans des groupes polaires (20), chaque groupe polaire (20) comprenant un ou plusieurs noeuds de stimulation (22, 24).

17. Système suivant la revendication 10, dans lequel chaque groupe polaire (20) est couplé de manière indépendante à un canal séparé du dispositif interactif (50).

18. Procédé destiné à simuler des sensations gustatives, comprenant :
a) le positionnement d'une plaque linguale (10) sur la langue (10) d'un utilisateur;
b) la réception, d'une source externe, d'une pluralité de signaux de stimulation électronique au niveau de ladite plaque linguale (10);
c) l'application de signaux de stimulation électronique reçus à une pluralité de noeuds de stimulation (13) disposés à l'intérieur de ladite plaque linguale (10) afin de stimuler par voie électronique des fibres nerveuses sensorielles (104) dans ladite langue d'utilisateur (100), et
d) la mesure d'un débit salivaire dudit utilisateur en réaction auxdits signaux de stimulation électronique appliqués.

19. Procédé suivant la revendication 18, dans lequel ladite pluralité de noeuds de stimulation (13) sont configurés de manière à être en contact avec des endroits prédéterminés d'une surface supérieure de la langue de l'utilisateur (100).

20. Procédé suivant la revendication 18, dans lequel l'action de réception (b) comprend en outre les actions suivantes :
la transmission, à partir d'un fournisseur de contenu (80), de données multimédia incluant des données de plaque linguale sur un réseau (110) à un dispositif interactif (50);
la conversion, au niveau dudit dispositif interactif (50), des données de plaque linguale transmises en lesdits signaux de stimulation électronique, et
la fourniture desdits signaux de stimulation électronique à ladite plaque linguale (10).
